(19) Europäisches Patentamt
European Patent Office
Office européen des brevets

(11) Veröffentlichungsnummer: **0 019 225**
**B1**

# (12) EUROPÄISCHE PATENTSCHRIFT

(45) Veröffentlichungstag der Patentschrift: **24.03.82**

(51) Int. Cl.3: **C 07 C 103/365**, C 07 C 103/38, C 07 C 102/00

(21) Anmeldenummer: **80102538.8**

(22) Anmeldetag: **08.05.80**

(54) **Verfahren zur Herstellung von N-Vinyl-N-alkyl-carbonsäureamiden.**

(30) Priorität: **16.05.79 DE 2919755**

(43) Veröffentlichungstag der Anmeldung: **26.11.80 Patentblatt 80/24**

(45) Bekanntmachung des Hinweises auf die Patenterteilung: **24.03.82 Patentblatt 82/12**

(84) Benannte Vertragsstaaten: **AT BE CH DE FR GB IT LI NL SE**

(56) Entgegenhaltungen:
**DE-A-1 445 737**
**DE-A-1 545 798**
**DE-A-2 113 338**
**DE-B-1 235 893**

(73) Patentinhaber: **HOECHST AKTIENGESELLSCHAFT, Postfach 80 03 20, D-6230 Frankfurt/Main 80 (DE)**

(72) Erfinder: **Jensen, Harald, Dr., Egerländer Strasse 37, D-6233 Kelkheim (Taunus) (DE)**
Erfinder: **Schmidt, Erwin, Dr., Am Flachsland 26, D-6233 Kelkheim (Taunus) (DE)**
Erfinder: **Mitzlaff, Michael, Dr., Berliner Strasse 26, D-6380 Bad Homburg (DE)**
Erfinder: **Cramer, Jürgen, Dr., Bergstrasse 27, D-6239 Eppstein/Taunus (DE)**
Erfinder: **Pistorius, Rudolf, Dr., Ausserhalb, D-6352 Ober-Mörlen (DE)**
Erfinder: **Pietsch, Hartmut, Dr., Kantstrasse 14, D-6238 Hofheim am Taunus (DE)**
Erfinder: **Dehmer, Klaus, Dr., Falkensteiner Strasse 20, D-6233 Kelkheim (Taunus) (DE)**

**EP 0 019 225 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

# Verfahren zur Herstellung von N-Vinyl-N-alkyl-carbonsäure-amiden

N-Vinyl-N-alkyl-carbonsäureamide sind wertvolle Zwischenprodukte und eignen sich insbesondere zur Herstellung von Homo- und Mischpolymerisaten mit interessanten und vielfältigen anwendungstechnischen Eigenschaften.

Zur Herstellung von N-Vinyl-N-alkyl-carbonsäureamiden ist eine Reihe verschiedener Methoden bekannt, welche im wesentlichen in 2 Gruppen eingeteilt werden können, nämlich in Methoden, welche von sekundären N-Alkyl-carbonsäureamiden (= N-Alkyl-carbonsäureamide, die am N noch ein H-Atom tragen) und Acetylen $C_2H_2$ ausgehen, und Methoden, bei welchen aus N-α-Alkoxyethyl-N-alkylcarbonsäureamiden Alkohol abgespalten wird.

A. Methoden, ausgehend von sekundären N-Alkyl-carbonsäureamiden und $C_2H_2$:

Die wohl ersten Arbeiten in dieser Richtung stammen von Reppe und Mitarb.; in Liebigs Ann. 601, 134 (1956) wird von diesen Autoren u.a. die Herstellung von N-Vinyl-N-methylacetamid durch Umsetzung von N-Methylacetamid mit $C_2H_2$ unter Zusatz von metallischem Kalium beschrieben. Nach 48-stündiger Umsetzung im Autoklaven bei 160–180 °C betrug die Ausbeute jedoch nur knapp 30% d.Th.

Das Verfahren wurde verbessert und weiterentwickelt durch Variation der Verfahrensbedingungen – teilweise in flüssiger (DH-PS 1 176 124), teilweise in der Gasphase (DE-PS 1 196 657), doch liessen sich auch hier keine befriedigenden Umsetzungsgrade erreichen, wenngleich die Ausbeuten an den gewünschten N-Vinyl-N-Alkylcarbonsäureamiden – bezogen auf das umgesetzte Ausgangsmaterial – relativ gut waren (80–90%).

Der Hauptnachteil dieser Verfahren auf Acetylenbasis besteht jedoch in der Notwendigkeit aufwendiger Sicherheitsmassnahmen wegen des nicht ungefährlich handzuhabenden Acetylens.

B. Alkoholabspaltung aus N-α-Alkoxyethyl-N-alkyl-carbonsäureamiden:

Nach dem Verfahren der DE-PS 1 235 893 erfolgt die Alkoholabspaltung aus N-α-Alkoxyethyl-N-alkyl-formamiden zu den entsprechenden N-Vinyl-N-alkyl-formamiden in Gegenwart schwach sauer wirkender oberflächenaktiver unlöslicher Substanzen oder von Mono-, Di-, Tri-, Oxy- oder Ketocarbonsäuren, die dem Reaktionsgemisch einen pH-Wert zwischen etwa 3 und 5,5 verleihen, durch Erhitzen auf Temperaturen zwischen 50 und 250 °C. Etwa bei der Herstellung von N-Vinyl-N-methyl-formamid aus N-α-methoxyethyl-N-methyl-formamid nach diesem Verfahren wird eine Ausbeute von 95%, bezogen auf umgesetztes Ausgangsmaterial (Umsatz 90,8%; s. Beispiel 1) erzielt.

Zur Herstellung der N-Vinyl- und N-Alkenyl-Derivate des Acetamids und der nächstfolgenden aliphatischen Säureamide sollen die entsprechenden N-α-Alkoxyalkylalkyl-carbonsäureamide in Gegenwart von Katalysatoren auf etwa 50 bis 180 bzw. 200 °C erhitzt werden (US-PS 3 377 340, GB-PS 1 125 324).

Nach dem Verfahren der US-PS soll dies in flüssiger Phase und in Gegenwart oberflächenaktiver unlöslicher Substanzen (Aluminiumoxid, Aluminiumphosphat, Zirkondioxid) erfolgen; das Verfahren der GB-PS arbeitet in der Gasphase in Gegenwart schwach saurer Katalysatoren, welche mit denen in der US-PS genannten teilweise identisch sind. Die in der GB-PS empfohlenen Katalysatoren sind die schwach sauren Oxide von Al, Be, Zr, W, die schwach sauren Phosphate von Ca, Al, Mo, B oder W, sowie Alumosilikate in der H-Form (Zeolithe etc.). Die Katalysatoren können auf einem inaktiven Trägermaterial wie Kieselgrur oder Aktivkohle etc. aufgebracht sein.

Die Ausbeuteangaben etwa für die Herstellung des N-Vinyl-N-methyl-acetamids liegen in den beiden genannten Patentschriften zwischen etwa 75 und 95% d.Th., bezogen auf umgesetztes Ausgangsmaterial.

Ebenfalls bei der Gruppe dieser Methoden genannt werden – wenngleich nicht voll dazugehörig – soll das Verfahren gemäss DE-PS 1 670 742, nach welchem N-Vinyl-N-alkyl-carbonsäureamide hergestellt werden durch Umsetzung von N-Alkylcarbonsäureamiden mit noch einem H-Atom am Amidstickstoff mit Acetaldehyd in Gegenwart von stark basischen oder nicht- bzw. schwerflüchtigen sauren Katalysatoren und Erhitzen des so erhaltenen Reaktionsgemisches in Gegenwart von nicht- bzw. schwerflüchtigen sauren Katalysatoren für maxiaml 60 Sek. auf eine Temperatur von 100–350 °C. Das Verfahren gehört deswegen nicht ganz in die Gruppe der Methoden zur Herstellung von N-Vinyl-N-alkyl-carbonsäureamiden durch Alkoholabspaltung aus N-α-Alkoxyethyl-N-alkylcarbonsäureamiden, weil hier kein N-α-Alkoxyethyl-N-alkyl-carbonsäureamid vorkommt. Man kann sich jedoch vorstellen, dass aus dem Ausgangsprodukt N-Alkyl-carbonsäureamid mit noch einem H-Atom am Amidstockstoff und Acetaldehyd ein den N-α-Alkoxyethyl-Derivaten der N-Alkylcarbonsäureamide analoges N-α-Hydroxyethyl-Derivat entsteht, aus welchem dann – wie bei den N-α-Alkoxyethyl-Derivaten durch Alkoholabspaltung – eben hier durch Wasserabspaltung die Vinylverbindung gebildet wird. Das Gelingen des Verfahrens hängt jedoch stark von der Einhaltung der ziemlich kritischen Reaktionsbedingungen ab; ausserdem sind Ausbeute und Umsetzungsgrad nicht immer ganz befriedigend (siehe etwa Beispiel 1: Herstellung von N-Vinyl-N-methyl-formamid aus N-Methyl-formamid und Acetaldehyd, Ausbeute 80% bei Umsetzungsgrad 49%).

Die Herstellung von sekundären N-Vinylcarbonsäureamiden, die am Amidstickstoff also noch 1 H-Atom tragen, ist etwa beschrieben in der DE-OS 23 36 977. Demnach werden N-α-Alkoxyethyl-carbonsäureamide im gasförmigen Zustand auf etwa 300–600 °C erhitzt und das dabei entstehende Gasgemisch wird unter rascher Abkühlung

kondensiert und aus dem Kondensat das N-Vinylcarbonsäureamid isoliert. Das Verfahren liefert gute Ausbeuten, ist jedoch auf die Herstellung sekundärer N-Vinyl-carbonsäureamide beschränkt.

Ausgangsprodukte für alle unter der Gruppe B genannten Verfahren (ausgenommen nur für das Verfahren gemäss DE-PS 1 670 742) sind N-α-Alkoxyalkyl-N-alkyl-carbonsäureamide und (im Falle des Verfahrens gemäss DE-OS 23 36 977) N-α-Alkoxyethyl-carbonsäureamide. Diese sind auf folgenden Wegen zugänglich.

C. Herstellung der N-α-Alkoxyalkyl-N-alkylcarbonsäureamide und N-α-Alkoxyethyl-carbonsäureamide:

N-α-Alkoxyalkyl-N-alkyl-carbonsäureamide sind etwa durch Umsetzung von sekundären N-Alkyl-carbonsäureamiden und Acetalen oder Halbacetalen erhältlich [Chem. Berichte 99, 2127 (1966); DE-PS 1 273 533]. Die Ausbeuten sind hier jedoch nicht immer befriedigend. So wird etwa in Beispiel 11 der DE-PS 1 273 533 (Umsetzung von N-Methylacetamid mit Acetaldehyd-Diethylacetal zu N-α-Ethoxyethyl-N-methylacetamid) nur eine Ausbeute von 26% angegeben.

Nach einem elektrochemischen Verfahren werden u.a. N-Alkyl- und N-Dialkyl-carbonsäureamide mit Alkoholen unter Verwendung bestimmter Leitsalze zu den N-α-Alkoxyalkyl-Derivaten alkoxyliert (DE-OS 21 13 338). Das Verfahren eignet sich jedoch weniger für die Herstellung von N-α-Alkoxyethyl-N-alkyl-carbonsäureamiden (weil bei 2 Alkylgruppen am Amidstickstoff die Alkoxylierung kaum selektiv nur an einer Alkylgruppe ansetzt und das entstehende Gemisch dann schwer auftrennbar ist), sondern hauptsächlich nur für die Herstellung sekundärer N-α-Alkoxyalkyl-carbonsäureamide mit noch einem H-Atom am Amidstickstoff.

Die elektrochemische Herstellung von sekundären N-α-Alkoxyethyl-carbonsäureamiden aus N-α-Carboxyethylcarbonsäureamiden ist beschrieben in der DE-OS 23 36 976; das Verfahren erfordert jedoch erst die Bereitstellung der als Ausgangsprodukte notwendigen N-α-Carboxyethylcarbonsäureamide, was einen gewissen Aufwand bedeutet.

Besser speziell zur Herstellung von sekundären N-Alkoxyethyl-carbonsäureamiden eignet sich das elektrochemische Verfahren der anodischen Alkoxylierung von sekundären N-Ethyl-carbonsäureamiden gemäss BE-PS 837 906; das Verfahren arbeitet mit einer gewissen Mindeststrommenge und ganz speziellen Leitsalzen.

Besonders günstig werden sekundäre N-Alkoxyethyl-carbonsäureamide hergestellt durch anodische Alkoxylierung von N-Ethyl-carbonsäureamiden mit einem Alkohol in einer Elektrolysezelle mit glasartigem Kohlenstoff als Anodenmaterial und mindestens einem Alkali- und/oder Tetraalkylammoniumalkosulfat als Leitsalz gemäss der gleichzeitig eingereichten Patentanmeldung P (HOE 79/F118).

Die N-α-Alkoxyethyl-N-alkyl-carbonsäureamide sind somit praktisch nur auf rein chemischem Weg (sekundäre Carbonsäureamide + Acetale oder Halbacetale) mit kaum befriedigenden Ausbeuten zugänglich. Der über diese Verbindungen führende Weg zu den entsprechenden N-Vinyl-N-alkyl-carbonsäureamiden ist also kaum ausreichend wirtschaftlich.

Andererseits sind die genannten elektrochemischen Verfahren, welche teilweise mit ausgezeichneten Ausbeuten verlaufen und auch sonst sehr vorteilhaft sind, praktisch nur für die Herstellung von sekundäuren N-α-Alkoxyethyl-carbonsäureamiden (mit noch einem Amid-Wasserstoff) geeignet. Die nachträgliche N-Alkylierung der chemisch empfindlichen und sterisch behinderten N-α-Alkoxyethyl-carbonsäureamide und auch der entsprechenden N-Vinyl-carbonsäureamide ist jedoch noch nicht beschrieben worden, obwohl N-Alkylierungen etwa von Carbonsäureaniliden und von Lactamen mit Alkylhalogeniden oder mit Dimethylsulfat in wässriger Alkalilauge bekannt sind (z.B. Synthesis 1976, 113–114, DE-OS 22 16 627, DE-OS 22 36 429, DD-PS 117 446, BE-PS 802 829).

Es war daher wünschenswert und bestand die Aufgabe, einen Weg zu finden, nach dem N-Vinyl-N-alkyl-carbonsäureamide auf eine wirtschaftlichere Weise als auf den bekannten Wegen hergestellt werden können.

Diese Aufgabe konnte erfindungsgemäss durch das im folgenden näher beschriebene neue 3-Stufenverfahren in befriedigender Weise gelöst werden.

Erfindungsgegenstand ist somit ein Verfahren zur Herstellung von N-Vinyl-N-alkyl-carbonsäureamiden, hauptsächlich solchen der Formel I

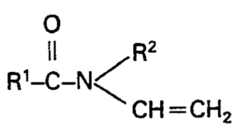

(I)

worin $R^1$=H, $CH_3$ oder $C_2H_5$, vorzugsweise H oder $CH_3$, insbesondere $CH_3$, und

$R^2$=$CH_3$ oder $C_2H_5$, vorzugsweise $CH_3$, ausgehend von N-Ethylcarbonsäureamiden der Formel II

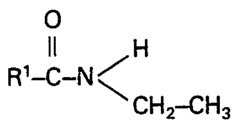

(II)

worin $R^1$ die gleiche Bedeutung wie in Formel I besitzt, das durch die folgenden Verfahrensschritte gekennzeichnet ist:

a) anodische Alkoxylierung der N-Ethylcarbonsäureamide der Formel II mit Alkoholen der Formel III

$$R^3OH \qquad (III)$$

worin $R^3$ = $(C_1$–$C_4)$-Alkyl, vorzugsweise $CH_3$ oder $C_2H_5$, insbesondere $CH_3$,

zu N-α-Alkoxyethylcarbonsäureamiden der Formel IV

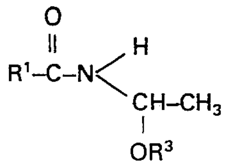 (IV)

worin $R^1$ und $R^3$ die gleiche Bedeutung wie in den Formeln I und III besitzen,

b) Alkylierung der N-α-Alkoxyethyl-carbonsäureamide IV durch Umsetzung mit einem Alkylierungsmittel der Formel V

$R^2X$ (V)

worin $R^2$ die gleiche Bedeutung wie in Formel I besitzt und

$X = Cl, Br, J$ oder $SO_4/2$,

in alkalischem Medium zu N-α-Alkoxyethyl-N-alkyl-carbonsäureamiden der Formel VI

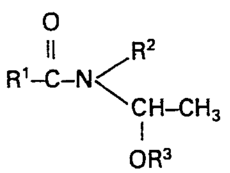 (VI)

worin $R^1$, $R^2$ und $R^3$ die bei den vorstehenden Formeln genannten Bedeutungen besitzen, und

c) Abspaltung von Alkohol der Formel III aus den N-α-Alkoxyethyl-N-alkyl-carbonsäureamiden VI durch Erhitzen auf Temperaturen von etwa 60 bis etwa 350 °C

oder anstelle der Stufen b und c im Anschluss an Stufe a

$b_1$) Abspaltung von Alkohol der Formel III aus den N-α-Alkoxyethyl-carbonsäureamiden IV zu N-Vinyl-carbonsäureamiden der Formel VII

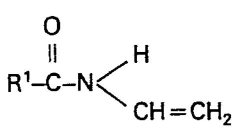 (VII)

worin $R^1$ die gleiche Bedeutung wie in Formel I besitzt, durch Erhitzen auf Temperaturen von etwa 60 bis etwa 600 °C und

$c_1$) Alkylierung der N-Vinyl-carbonsäureamide VII durch Umsetzung mit einem Alkylierungsmittel der Formel V in alkalischem Medium.

Das Verfahren geht von einfachen, leicht zugänglichen Ausgangsprodukten aus (N-Ethylcarbonsäureamide II) und verläuft ausserordentlich glatt und mit hohen Ausbeuten, welche bei sorgfältiger Verfahrensführung bei über 90% d.Th. liegen.

Die Kombination der 3 Verfahrensschritte

a) Anodische Alkoxylierung +
b) Alkylierung +
c) Alkoholabspaltung bzw.
a) Anodische Alkoxylierung +
$b_1$) Alkoholabspaltung +
$c_1$) Alkylierung

war durch nichts nahegelegt; denn trotz des Bedürfnisses an einem wirtschaftlichen Verfahren zur Herstellung von N-Vinyl-N-alkyl-carbonsäureamiden und trotz der schon einige Zeit bekannten anodischen Alkoxylierung von N-Ethylcarbonsäureamiden, der altbekannten Alkylierung von N-Verbindungen (wenngleich von sekundären N-α-Alkoxyethyl- und N-Vinyl-carbonsäureamiden) sowie der ebenfalls schon etliche Jahre bekannten Alkoholabspaltung aus N-α-Alkoxyethyl-carbonsäureamiden wurden die 3 fraglichen Verfahrensschritte noch nicht früher zu einem einheitlichen und wirtschaftlichen Gesamtverfahren kombiniert. Es war auch nicht naheliegend, die an sich bekannten Methoden zur Alkylierung von N-Verbindungen und insbesondere von einigen Carbonsäureaniliden und Lactamen in alkalischem Medium auf sekundäre N-α-Alkoxyethyl- und N-Vinyl-carbonsäureamide zu übertragen, weil diese speziellen sekundären Carbonsäureamide in alkalischem Medium erheblich instabiler sind als etwa Carbonsäureanilide und Lactame und etwa auch N-α-Alkoxyethyl-N-alkyl- und N-Vinyl-N-alkyl-carbonsäureamide.

Ausgangsstoffe für das erfindungsgemässe 3-Stufenverfahren sind N-Ethyl-carbonsäureamide der vorstehenden Formel II (N-Ethyl-formamid, -acetamid und -propionamid); bevorzugte Ausgangsprodukte sind N-Ethyl-formamid und -acetamid, insbesondere N-Ethyl-acetamid. Diese Verbindungen sind auf bekannte Weise leicht zugänglich, z.B. aus den freien Carbonsäuren oder deren Estern und Ethylamin.

Die Ausgangs-N-ethyl-carbonsäureamide II werden dann in der ersten Verfahrensstufe,

a) der anodischen Alkoxylierung, mit Alkoholen der Formel III[($C_1$–$C_4$)-Alkanole, vorzugsweise Methanol oder Ethanol, insbesondere Methanol] elektrolysiert. Dies geschieht vorteilhaft nach den Verfahren gemäss DE-OS 21 13 338 oder BE-PS 837 906. Besonders günstig ist die anodische Alkoxylierung nach dem Verfahren der gleichzeitig eingereichten Patentanmeldung EP-OS 19 226 in einer Elektrolysezelle mit einer Anode aus glasartigem Kohlenstoff und mindestens einem Alkali und/oder Tetraalkylammoniumalkosulfat als Leitsalz; bevorzugte Leitsalze sind Na-, K- sowie Tetramethylammoniummetho- und Ethosulfate, insbesondere Tetramethylammoniummethosulfat.

Als Kathodenmaterialien können die hierfür üblichen Nichtedelmetalle wie Stahl, Nickel etc. verwendet werden. Es ist sowohl der Einsatz eines einzigen Leitsalzes als auch einer Leitsalz-Mischung möglich.

Die Elektrolyse-Ausgangslösung enthält das Ausgangs-N-Ethylcarbonsäureamid und das Leitsalz in alkoholischer Lösung. Dabei ist es günstig, wenn das Mol-Verhältnis von N-Ethyl-carbonsäureamid zu Alkohol zwischen etwa 1:1 und etwa 1:100, vorzugsweise zwischen etwa 1:2 und etwa 1:60, und insbesondere zwischen etwa 1:5 und etwa 1:50 beträgt.

Die Konzentration der (Gesamt)-Elektrolyselösung an Leitsalz beträgt zweckmässig zwischen etwa 0,1 und etwa 40 Gew.-%, vorzugsweise zwischen etwa 5 und etwa 20 Gew.-%.

Die Zugabe des Leitsalzes erfolgt meist nach dem Herstellen der alkoholischen Lösung, doch kann die Reihenfolge auch geändert werden.

Wasser muss von der Elektrolyse nicht vollständig ausgeschlossen werden, da geringe Feuchtigkeitsanteile den Reaktionsablauf kaum beeinträchtigen. Bei dem Elektrolyse-Verfahren gemäss der erwähnten gleichzeitig eingereichten Patentanmeldung wird vorteilhaft eine Strommenge von mindestens etwa 2,5 Faraday/Mol. Ausgangsverbindung verwendet. Geringere Strommengen sind möglich, sie verringern jedoch den Umsatz von Carbonsäureamiden II. Die Stromdichte wird zweckmässig zwischen etwa 10 und 1000 mA/$cm^2$, vorzugsweise zwischen etwa 20 und 600 mA/$cm^2$, eingestellt. Niedrigere Stromdichten sind möglich, jedoch ohne Vorteil; sie verlangsamen vielmehr die Produktbildung.

Als Arbeitstemperatur wird für diese Elektrolyse vorteilhaft eine Temperatur gewählt, die unterhalb der Siedetemperatur des jeweiligen Alkohols und oberhalb der Schmelztemperatur der Elektrolyselösung liegt. Im allgemeinen werden Temperaturen von etwa −10 bis +100 °C vorzugsweise von etwa 0 bis 60 °C, angewandt.

Normalerweise wird die Elektrolyse bei Atmosphärendruck durchgeführt, doch ist – wenngleich ohne Vorteile – auch die Durchführung unter vermindertem oder erhöhtem Druck möglich.

Zur Vermeidung von explosiblen Gasgemischen aus (bei der Elektrolyse entstehendem) Wasserstoff und Luft ist das Arbeiten unter Zusatz eines Inertgases wie z.B. Stickstoff vorteilhaft.

Das Verfahren kann sowohl kontinuierlich als auch diskontinuierlich betrieben werden. Es zeichnet sich durch den besonderen Vorteil aus, dass – entgegen der auch bei Elektroden aus glasartigem Kohlenstoff bekannten Materialabtragung (siehe N.L. Weinberg «Technique of Electroorganic Synthesis», Vol. 5, Part I, S. 19, Abs. 2; John Wiley Verlag 1972) – in dem hier verwendeten Elektrolytsystem praktisch kein Abtrag eintritt. Ausserdem bildet sich an der Kathode kein störender schwerlöslicher Niederschlag, der bei Verwendung von F-haltigen Leitsalzen oft zu beobachten ist.

Wegen der höheren Löslichkeit der bei diesem Elektrolyseverfahren verwendeten Leitsalze (Alkosulfate) in Alkoholen können schliesslich auch erheblich höhere Stromdichten angewandt werden als bei den bekannten Verfahren des Standes der Technik, welche hauptsächlich mit den in Alkoholen weniger löslichen Tetrafluoroboraten, Hexafluorophosphaten und Nitraten arbeiten; dadurch sind in kürzerer Zeit höhere Umsätze möglich.

b) Alkylierung der in Stufe a) erhaltenen N-α-Alkoxyethyl-carbonsäureamide IV:

Als Alkylierungsmittel können an sich alle möglichen für die N-Alkylierung von Carbonsäureamiden bekannten Agenzien verwendet werden, doch haben sich als zweckmässigste Alkylierungsmittel solche der Formel

$$R^2X \qquad (V)$$

erwiesen,
worin $R^2$, die Methyl- oder Ethylgruppe, vorzugsweise die Methylgruppe bedeutet und
X=Cl, Br, J oder die halbe Sulfatgruppe ist.
Beispielhafte Alkylierungsmittel sind:
Chlormethan, Brommethan, Jodmethan, Dimethylsulfat, Chloräthan, Jodäthan etc.; bevorzugtes Alkylierungsmittel ist Chlormethan.

Das Alkylierungsmittel wird im Verhältnis zum zu alkylierenden N-α-Alkoxyethyl-carbonsäureamid IV zweckmässig in etwa äquimolarer Menge bis zu einem etwa 100 (Mol)-%igen Überschuss eingesetzt. Ein höherer Überschuss verlangsamt die Alkylierung und führt zu Verlusten an Alkylierungsmittel und Alkali durch Verseifung. Bei einem Unterschuss an Alkylierungsmittel geht N-α-Alkoxyethyl-carbonsäureamid IV durch Nebenreaktionen verloren.

Das Verfahren ist weiterhin zweckmässig so zu führen, dass das N-α-Alkoxyethyl-carbonsäureamid IV immer einem Überschuss sowohl von Alkylierungsmittel als auch von Alkali gegenübersteht. Dabei ist es sehr wohl möglich, dass das Gesamtverhältnis von Alkylierungsmittel zu Amid IV nicht höher als äquimolar ist; in diesem Fall wäre das Verfahren so zu führen, dass das Amid IV und Alkali in vorgelegtes Alkylierungsmittel eindosiert werden.

Das alkalische Medium während der Alkylierungsreaktion wird vorteilhaft durch ein Alkalihydroxyd, insbesondere NaOH und/oder KOH bewirkt. Besonders bevorzugt ist NaOH.

Das Alkalihydroxyd kann in den handelsüblichen Formen als Pulver, Plätzchen, Schuppen etc. oder auch als etwa 50%ige wässrige Lösung eingesetzt werden. Die Alkalihydroxydmenge ist so zu berechnen, dass die Konzentration des Alkalihydroxyds am Ende der Reaktion noch mindestens 20% beträgt, da sonst kaum mehr Alkylierung eintritt. Bei Verwendung von festem Alkalihydroxyd ist ein etwa 40–60%iger Überschuss zweckmässig. Bei geringerem Alkaliüberschuss bleibt in zunehmendem Mass nicht alkyliertes Ausgangsamid IV zurück. Wenn man mit festem Alkalihydroxyd und/oder unter Zusatz von Lösungsmitteln arbeitet, so wird die Reaktion durch den Zusatz von Phasentransferkatalysatoren deutlich beschleunigt. Als solche sind z.B. quartäre Ammonium- und Phosphonium-Salze sowie Sulfonium-Salze geeignet, wie z.B. Benzyl-triäthylammoniunchlorid, Tetrabutylammoniumsulfat, Tetramethylphosphoniumbromid etc. Bevorzugt sind die quartären Ammoniumsalze, insbesondere Tetramethyl- und Tetraethylammoniumchlorid und -sulfat, die auch noch den Vorteil haben, wie Ammoniumsalze allgemein, die nachfolgende Alkoholabspaltung bereits bei niedrigen Temperaturen (etwa 60 bis 70 °C) zu katalysieren. Deswegen können in diesem Fall im Prinzip Alkylierungsreaktion b) und Alkoholabspaltung c) in einem Reaktionsgefäss vereinigt werden.

Bei Verwendung von festem Alkalihydroxyd ist es zweckmässig, dieses vor Beginn der Reaktion

mit einem Verdünnungsmittel aufzuschlämmen, als welches sich besonders das Alkylierungs-Endprodukt selbst eignet. Andere Verdünnungsmittel sind z.B. Methylenchlorid, Toluol, Xylol etc., die auch zum Auswaschen des Reaktionsgefässes und des Filterkuchens geeignet sind.

Die Alkylierung wird zweckmässig in dem Temperaturbereich zwischen etwa +10 und etwa +60 °C durchgeführt, doch ist die Reaktion nicht auf diesen Temperaturbereich begrenzt. Unterhalb von etwa 10 °C wird die eingesetzte Alkalilauge bzw. das alkalische Medium zunehmend viskoser und die Reaktion verläuft zunehmend langsamer. Oberhalb etwa +60 °C beginnt die Ausbeute am Alkylierungsprodukt langsam abzusinken und es wird zunehmend Alkylierungsmittel durch Nebenreaktionen verbraucht.

Um die Alkylierung in etwa 3 bis 6 Stunden zu Ende zu führen, alkyliert man vorteilhaft in etwa 50%iger wässriger Natronlauge bei etwa 20 bis etwa 40 °C, bei Verwendung von festem Alkalihydroxyd bei etwa 30–60 °C.

Der Alkylierungsansatz wird auf übliche Weise aufgearbeitet. Z.B. wird die organische Phase bei Verwendung wässriger Natronlauge durch Dekantieren oder Zentrifugieren abgetrennt. Bei Verwendung festen Alkalis kann das Reaktionsprodukt durch Absaugen isoliert werden. Die anorganischen Rückstände können mit organischen Lösungsmitteln (z.B. Toluol) extrahiert werden. Das Reaktionsprodukt wird dann, ggf. nach Abziehen des Lösungsmittels, unter normalem oder zweckmässig vermindertem Druck destilliert.

c) Abspaltung von Alkohol III aus den in Stufe b) erhaltenen N-Alkoxyethyl-N-alkyl-carbonsäureamiden VI: Die Alkoholabspaltung aus den Produkten VI wird – ohne oder mit Katalysatoren – im Temperaturbereich von etwa 60 – etwa 350 °C, vorzugsweise von etwa 180–350 °C, durchgeführt. Im unteren Teil dieses Temperaturbereichs kann dabei in flüssiger Phase gearbeitet werden, während bei höherer Temperatur praktisch nur die Gasphasenreaktion in Frage kommt. Als Katalysatoren können praktisch alle für die Alkoholabspaltung von N-α-Alkoxyalkyl-N-alkyl-carbonsäureamiden zu den entsprechenden Vinylverbindungen bekannten Substanzen verwendet werden, wie z.B. die schwachsauren Oxyde von Al, Be, Zr, W, die schwachsauren Phosphate von Ca, Al, Mo, B, W oder Alumosilikate in der H-Form (Zeolithe etc.), ggf. auf Trägermaterialien wie Kieselgur, Aktivkohle etc. (US-PS 3 377 340, GB-PS 1 125 324). Günstige Katalysatoren sind auch Ammoniumsalze, vor allem ($C_1$–$C_4$)-Alkylammoniumhalogenide-sulfate und -phosphate.

Auch was die übrigen Bedingungen der Spaltungsreaktion (verminderter Druck, Normaldruck etc.) und die apparative Durchführung anbetrifft, kann auf den bekannten Stand der Technik verwiesen werden.

Anstelle der Reaktionsstufenfolge: anodische Alkoxylierung a-Alkylierung b-Alkoholabspaltung c kann auch auf die anodische Alkoxylierung a die Alkoholabspaltung $b_1$ aus den N-α-Alkoxyethyl-carbonsäureamiden IV zu den N-Vinyl-carbonsäureamiden VII und deren Alkylierung $c_1$ zu den gewünschten Endprodukten erfolgen. Die Schrittfolge a-b-c ist allerdings gegenüber der Folge a-$b_1$-$c_1$ bevorzugt.

Die Alkoholabspaltung $b_1$ aus den Verbindungen IV erfolgt analog wie die Alkoholabspaltung in Stufe c aus den entsprechenden N-α-Alkoxyethyl-N-alkyl-carbonsäureamiden VI. Der bevorzugte Temperaturbereich dieser Spaltung liegt aber höher als bei c, nämlich bei ca. 180–600 °C. Ebenso gilt für die Alkylierung der N-Vinylcarbonsäureamide VII nach Stufe $c_1$ das gleiche wie für die Alkylierung der N-α-Alkoxyethyl-carbonsäureamide IV in Stufe b.

Sämtliche Stufen des erfindungsgemässen Kombinationsverfahrens verlaufen einfach und mit ausgezeichneten Ausbeuten, so dass auch die Gesamtausbeuten des Verfahrens in der Regel über 90% d.Th., bezogen auf die Ausgangs-N-Ethyl-carbonsäureamide II, liegen, und zwar unabhängig davon, ob das Verfahren nun in der Reaktionsfolge a-b-c oder a-$b_1$-$c_1$ durchgeführt wird. Das Verfahren stellt daher einen erheblichen technischen und wirtschaftlichen Fortschritt auf dem Gebiet der Herstellung von N-Vinyl-N-alkyl-carbonsäureamiden, insbesondere des N-Vinyl-N-methylacetamids, dar.

Die Erfindung wird nun anhand der folgenden Beispiele näher erläutert.

a) Anodische Alkoxylierung

Es werden hier nur Beispiele für die für das erfindungsgemässe Verfahren bevorzugte Ausführungsform der anodischen Alkoxylierung gemäss der gleichzeitig eingereichten Anmeldung P (HOE 79/F118) gebracht, da die anodische Alkoxylierung ansonsten Stand der Technik ist (DE-OS 21 13 338, BE-PS 837 906 etc.).

Beispiel 1–7

In einer Elektrolysezelle gemäss der Figur der DE-OS 2 113 338 von etwa 500 ml Inhalt mit Dekkel und Rückflusskühler wird eine Mischung aus dem jeweiligen Carbonsäureamid und dem entsprechenden Alkohol eingefüllt, in der das Leitsalz gelöst wird. Je eine Platte aus Stahl und glasartigem Kohlenstoff (Breite × Länge = 50×130 $mm^2$) werden so angeordnet, dass sie einen gegenseitigen Abstand von 3 bis 5 mm haben und 100 mm in die Lösung eintauchen. Der Zelleninhalt wird während der Elektrolyse mit Hilfe eines Magnetrührers mit 50 bis 60 Umdrehungen pro Minute umgerührt und dabei auf dem in folgender Tabelle 1 jeweils angegebenem Wert gehalten. Nachdem die (ebenfalls in Tabelle 1 angegebene) Strommenge Q durchgeleitet worden ist, wird der Strom abgeschaltet.

Die Elektrolyselösung wird in bekannter Weise aufgearbeitet.

Die Ergebnisse für die Beispiele 1 bis 7 sind in folgender Tabelle 1 zusammengefasst:

Tabelle 1

| Beispiel Nr. | Amid $R^1CONHC_2H_5$ $R^1$ | [g] | Alkohol ROH R' | [g] | Leitsalz | [g] | Elektrolysedaten $\bar{J}$ | $\bar{U}$ | Q | T | Produkt | Ausbeute M | S |
|---|---|---|---|---|---|---|---|---|---|---|---|---|---|
| 1 | H | 100 | $CH_3$ | 250 | Tetramethylammoniummethosulfat | 50 | 50 | 12,3 | 3,0 | 45 | N-(α-Methoxyethyl)-formamid | 74,1 | 49,4 |
| 2 | H | 100 | $CH_3$ | 250 | Natriummethosulfat | 15 | 45 | 29,9 | 3,8 | 20 | N-(α-Methoxyethyl)-formamid | 72,0 | 37,9 |
| 3 | H | 100 | $CH_3$ | 250 | Kaliummethosulfat | 9 | 45 | 50,1 | 3,1 | 10 | N-α-Methoxyethyl)-formamid | 70,9 | 45,7 |
| 4 | $CH_3$ | 100 | $C_2H_5$ | 263 | Tetramethylammoniummethosulfat | 50 | 30 | 47,3 | 4,2 | 10 | N-α-Ethoxyethyl)-acetamid | 52,1 | 24,8 |
| 5 | $CH_3$ | 100 | $C_2H_5$ | 263 | Tetraethylammoniu m-ethosulfat | 60 | 30 | 29,2 | 4,8 | 30 | N-(α-Ethoxyethyl)-acetamid | 51,3 | 21,4 |
| 6 | $CH_3$ | 90 | n-$C_4H_9$ | 300 | Tetramethylammoniummethosulfat | 30 | 20 | 49,2 | 4,8 | 30 | N-(α-n-Butoxyethyl)-acetamid | 45,1 | 18,8 |
| 7 | $C_2H_5$ | 60 | $CH_3$ | 300 | Tetramethylammoniummethosulfat | 45 | 20 | 19,9 | 4,0 | 20 | N-α-Methoxyethyl)-propionamid | 80,3 | 40,2 |

$\bar{J}$ = Stromdichte [mA/cm$^2$]
$\bar{U}$ = mittlere Zellspannung [V]
Q = Strommenge [Faraday/Mol Amid]
T = Zelltemperatur [°C]
M = Materialausbeute [% d.Th.]
S = Stromausbeute [%]

Beispiel 8–10

In einer Durchflussapparatur mit Umwälzpumpe, Wärmetauscher und Entgasungsgefäss wird eine ungeteilte Elektrolysezelle mit einer blockartigen Elektrodenkombination eingebaut. Diese besteht aus einer Anode aus glasartigem Kohlenstoff, aus einer Kathode aus Stahl und aus dazwischen vier bipolar geschalteten Elektrodenplatten aus glasartigem Kohlenstoff. Zwischen diese Platten wird jeweils ein Stapel aus Nickelgewebe (2 Lagen der Maschenweite 0,19 mm und 0,1 mm Drahtstärke und dazwischen zwei Lagen der Maschenweite 0,5 mm und 0,3 mm Drahtstärke) und aus Polyethylengewebe (1 Lage der Maschenweite 0,9 mm und 0,3 mm Fadenstärke) so eingeschoben, dass das Nickelgewebe auf die Kathodenseiten der Kohleplatten bzw. die Stahlplatte zu liegen kam. Diese Kombinationen wird zur Minimierung – des Elektrodenabstandes zusammengepresst eingebaut. Alle Elektrodenplatten sind mit einem Polyethylenrahmen eingefasst, der senkrecht zur Strömungsrichtung des Elektrolyten 22 mm breit – parallel zur Strömungsrichtung 12 mm breit und wie jede der Platten etwa 2,5 mm dick war. Die wirksame Elektrodenfläche jeder Anode betrug 255 cm$^2$.

Die in dieser Apparatur durchgeführten Beispiele sind in der folgenden Tabelle 2 zusammengefasst:

Tabelle 2

| Beispiel Nr. | Amid $CH_3CONHC_2H_5$ | | R'OH | | Leitsalz | | Elektrolysedaten | | | | Produkt | Ausbeute | |
|---|---|---|---|---|---|---|---|---|---|---|---|---|---|
| | | [g] | R' | [g] | | [g] | J̄ | Ū | Q | T | | M | S |
| 8 | do. | 4830 | $CH_3$ | 6485 | Tetramethylammoniummethosulfat | 2485 | 157 | 30,1 | 2,8 | 48 | N-(α-Methoxyethyl)-acetamid | 86,5 | 61,8 |
| 9 | do. | 2000 | do. | 10000 | do. | 3000 | 179 | 25,8 | 3,0 | 37 | do. | 89,3 | 59,5 |
| 10 | do. | 6000 | do. | 1000 | do. | 3750 | 153 | 26,3 | 2,6 | 46 | do. | 91,9 | 70,7 |

Erklärung der Symbole vgl. Tabelle 1

b) Alkylierung

Beispiel
N-α-Methoxyethyl-N-methyl-formamid

Zu einer auf 5–10 °C gekühlten Mischung von 160 g 50%iger Natronlauge und 31,5 g (0,25 Mol) Dimethylsulfat tropft man unter intensivem Rühren innerhalb 30 Minuten 20,6 g (0,2 Mol) N-α-Methoxyethyl-formamid. Man rührt bei der gleichen Temperatur 30 Min. nach, zersetzt überschüssiges Dimethylsulfat durch Zusatz wässrigen Ammoniaks, extrahiert mit i-Propylether und destilliert nach Abtreiben des Lösungsmittels i.V. Man erhält 21,3 g (91% d.Th.) N-α-Methoxyethyl-N-methyl-formamid vom $Kp_{17}$ 80–83 °C.

Beispiel 2
N-α-Ethoxyethyl-N-methyl-acetamid

Bei 10–15 °C tropft man zu 100 ml 50%iger Natronlauge gleichzeitig unter intensivem Rühren 37,8 g (0,3 Mol) Dimethylsulfat und 26,2 g (0,2 Mol) N-α-Ethoxyethyl-acetamid.

Man rührt 3 Stunden nach, zersetzt restliches Dimethylsulfat durch Zusatz von konz. Ammoniak, trennt im Scheidetrichter, extrahiert die wässrige Schicht mehrmals mit Toluol und destilliert nach Abtreiben des Lösungsmittels i.V. Man erhält 26,1 g (90% d.Th.) N-α-Ethoxyethyl-N-methyl-acetamid vom $Kp_{24}$ 90–94 °C.

Beispiel 3
N-α-Methoxyethyl-N-ethyl-acetamid

Zu einer Mischung von 117 g (1 Mol) N-α-Methoxyethyl-acetamid, 500 ml wasserfreiem Dimethylsulfoxid und 70 g (1,25 Mol) gepulvertem Kaliumhydroxid tropft man unter Rühren bei 25 °C 136 g (1,25 Mol) Bromethan. 2 Stunden nach beendetem Eintropfen extrahiert man gründlich mit Hexan. Nach Abtreiben des Lösungsmittels wird das Reaktionsprodukt i.V. fraktioniert. Man erhält 50 g (34.5% d.Th.) N-α-Methoxyethyl-N-ethyl-acetamid vom $Kp_{16}$ 83–85 °C.
$^1$H-NMR ($CDCl_3$): 1,0–1,45 (m), 2,1 (s) 3,1–3,45 (m); 4,8–5,1 (q); 5,65–6,0 (q) ppm.

Beispiel 4
N-α-Methoxyethyl-N-ethyl-acetamid

Zu 300 ml 50%iger Natronlauge tropft man bei 10 °C gleichzeitig 58,5 g (0,5 Mol) N-α-Methoxyethyl-acetamid und 84,2 g (0,55 Mol) Jodethan. Zwei Stunden nach beendeter Zugabe trennt man im Scheidetrichter, extrahiert den wässrigen Anteil mit Hexan und destilliert nach Abtreiben des Lösungsmittels i.V. Man erhält 37 g (51% d.Th.) N-α-Methoxyethyl-N-ethyl-acetamid vom $Kp_{16}$ 83–85 °C.
$^1$H-NMR ($CDCl_3$): 1,0–1,45 (m); 2,1 (s) 3,1–3,45 (m) 4,8–5,1 (q); 5,65–6,0 (q) ppm.

Beispiel 5
N-α-Methoxy-ethyl-N-methylacetamid

Man versetzt 500 ml 50%ige Natronlauge bei 5–10 °C mit 25 g (0,2 Mol) Dimethylsulfat und tropft unter Rühren innerhalb von 3 Stunden gleichzeitig 158 g Dimethylsulfat (1,25 Mol) und 117 g (1,0 Mol) N-α-Methoxyethyl-acetamid zu. Nach weiteren 3 Stunden versetzt man mit 50 ml konz. Ammoniak, trennt nach 30 Min. im Scheidetrichter und extrahiert die wässrige Schicht mit Diethylether. Nach Abtreiben des Lösungsmittels

erhält man durch Destillation i.V. 120 g (92% d.Th.) N-α-Methoxyethyl-N-methyl-acetamid vom $Kp_{18}$ (76–81 °C.
$^1$H–NMR ($CDCl_3$): 1,2 (d); 1,35 (d) 1,05 (d); 1,25 (d); 3,15 (d) 4,85–5,15 (q); 5,6–5,9 (q), ppm.

Beispiel 6
N-α-Methoxyethyl-N-methyl-acetamid

In einem mit Ankerrührer versehenen Druckkessel legt man 1800 g 50%ige Natronlauge, 900 g schuppenförmiges Natriumhydroxid und 300 g Chlormethan vor. Innerhalb von 3 Stunden dosiert man 1760 g N-α-Methoxyethyl-acetamid und 1000 g Chlormethan gleichmässig zu. Nach weiteren 3 Stunden wird der Kessel entspannt und der Inhalt durch Einleiten von Stickstoff entgast. Man trennt das spezifisch leichtere Reaktionsprodukt in einer Zentrifuge von spezifisch schwereren anorganischen Bestandteilen, dekantiert und destilliert das Reaktionsprodukt i.V. Man erhält 1804 g (91% d.Th.) N-α-Methoxyethyl-N-methylacetamid. vom $Kp_{1,0}$ 48 °.

Durch Variation der Reaktionstemperatur unter sonst gleichen Bedingungen ergeben sich folgende Ausbeuten:

| Reaktions-temp. ( °C) | Ausbeute (%) | Rückgewonnenes Ausgangs-material |
|---|---|---|
| 10 | 50 | 40 |
| 15 | 81 | 7,5 |
| 20 | 92 | 2,5 |
| 25 | 83 | – |
| 30 | 83 | – |
| 40 | 81 | – |
| 50 | 70 | – |

Beispiel 7
N–α-Methoxyethyl-N-methyl-acetamid

In einem mit Ankerrührer versehenen Druckkessel werden

525 g N-α-Methoxyethyl-N-methylacetamid als Verdünnungsmittel,
700 g (17,5 Mol) Natriumhydroxid in Schuppenform,
30 g Wasser und
100 g Chlormethan (2 Mol) bei 40 ° vorgelegt. Unter Wasserkühlung fördert man innerhalb von 3 Stunden gleichzeitig
1170 g (10 Mol) N-α-Methoxyethyl-acetamid und
525 g Chlormethan (10,5 Mol) in den Kessel. Nach beendeter Förderung lässt man 3 Stunden bei 40 ° rühren.

Der Kesselinhalt wird über ein Polyesterfilter abgedrückt. Kessel und Nutschkuchen werden mit Methylenchlorid nachgewaschen. Nach Abtreiben des Lösungsmittels erhält man aus dem Filtrat durch Destillation i.V. 1717 g N-α-Methoxyethyl-N-methylacetamid (91% d.Th. nach Abzug des als Verdünnungsmittel eingesetzten Produktes) vom $Kp_{1,5}$ 49–51 °, und 33 g (3% d.Th.) nicht methylierten Ausgangsmaterial.

Führt man die Reaktion unter sonst gleichen Versuchsbedingungen durch, so erhält man folgende Ausbeute.

| Reaktions-temperatur ( °C) | 20 | 30 | 40 | 50 | 60 |
|---|---|---|---|---|---|
| Ausbeute Endprod. (%) | 74 | 83 | 90 | 92 | 93 |
| Rückgew. Ausgangsmat. (%) | 9 | 7 | 3 | 3 | 6 |

Beispiel 8
α-Methoxyethyl-N-methyl-acetamid

Man verfährt wie in Beispiel 7 bei 20 °, setzt aber unter sonst gleichen Reaktionsbedingungen dem Gemisch von Natriumhydroxid und N-α-Methoxyethyl-N-methylacetamid vor dem Zudosieren der übrigen Reaktionskomponenten anstelle von 30 g Wasser 50 g eines $C_{12}/C_{18}$-Alkyl-dimethylbenzylammoniumchlorids, 50%ig in Wasser, zu. Man erhält bereits bei 20 °-N-α-Methoxyethyl-α-N-methylacetamid in einer Ausbeute von 92%.

Das Rohprodukt wird zur Rektifizierung unter 1 Torr über einen Dünnschichtverdampfer in einer kontinuierlichen Kolonne destilliert. Am Kopf der Kolonne nimmt man N-α-Methoxyethyl-N-methylacetamid ab (Ausbeute 92%), am Fusse der Kolonne nicht methyliertes Ausgangsmaterial (5%).

c) Alkoholabspaltung

Beispiel 1

In einem Dünnschichtverdampfer wird unter Stickstoffüberlagerung bei 270 ° N-α-Methoxyethyl-N-methylacetamid verdampft. Der Dampf wird durch den mit einer porösen Kieselsäure als Katalysator gefüllten Ofen geleitet. Die Spaltprodukte werden am Ende des Ofens kondensiert. Tabelle 1 zeigt den Gehalt an nichtgespaltenem Ausgangsmaterial im Kondensat in Abhängigkeit von der Ofentemperatur bei einem Durchsatz von 400 g Ausgangsmaterial/Stunde.

| Temp. ( °C) | N-α-Methoxyethyl-N-methyl acetamid, Restgehalt (%) |
|---|---|
| 225 | 3,3 |
| 240 | 2,0 |
| 250 | 1,7 |
| 265 | 0,4 |
| 280 | 0,3 |
| 290 | 0,3 |

Die Gesamtausbeuten an N-Vinyl-N-methylacetamid und nicht gespaltenem Ausgangsmaterial liegen bei 97–98%.

Das rohe Spaltmaterial wird in einer Füllkörperkolonne rektifiziert. Nach Abdestillieren des abgespaltenen Methanols erhält man in quant. Ausbeute N-Vinyl-N-methyl-acetamid vom $Kp_{11}$ 51,5 °C.
$^1$H–NMR ($CDCl_3$); 2,15 (s); 3,05 (s) 4,2–4,6 (d,d); 6,2–6,8 (m).

Beispiel 2

23,4 g (o,2 Mol) N-α-Methoxyethyl-N-methylformamid werden unter Stickstoff in 1 Stunde aus einem auf 300 °C geheizten 50 ml-Kolben flashdestilliert in ein mit poröser Kieselsäure als Katalysator gefülltes Quarzrohr von 14 mm Durchmesser, das auf eine Länge von 16 cm in einem Röhrenofen auf 350 °C geheizt wird. Die Spaltprodukte werden in einer eisgekühlten Vorlage gesammelt.

Nach Abdestillieren des gebildeten Methanols erhält man durch Vakuumdestillation 15 g (88% d.Th.). N-Vinyl-N-methylformamid vom $Kp_{12}$ 42–45 °C.
$^1$H–NMR ($CDCl_3$) ppm 3,05 (d);
4,3–4,7 (m); 4,5–4,95 (dd);
7,1–7,6 (m); 8,1 (s)
8,3 (s).ppm.

Beispiel 3

28,9 g (0,2 Mol) N-α-Methoxyethyl-N-ethylacetamid werden innerhalb 1 Stunde aus einem auf 300 °C geheizten 50 ml-Kolben unter Stickstoff flash-destilliert in eine mit dem gleichen Katalysator wie in den vorhergehenden Beispielen gefülltes Quarzrohr von 14 mm Durchmesser, das auf eine Länge von 16 cm in einem Röhrenofen auf 300 °C geheizt wird. Die Spaltprodukte werden in einer eisgekühlten Vorlage gesammelt.

Nach Abdestillieren des gebildeten Methanols erhält man durch Vakuumdestillation 20,5 g (92% d.Th.) N-Vinyl-N-ethyl-acetamid vom $Kp_{13}$ 62 °C.
$^1$H–NMR ($CDCl_3$); 1,0–1,2 (t);
2,2 (s); 3,5–3,9 (q); 4,2–4,6 (d,d);
6,6–8,5 (m) ppm.

Beispiel 4

28,9 g (0,2 Mol) N-α-Ethoxyethyl-N-methylacetamid werden innerhalb 1 Stunde aus einem auf 300 °C geheizten 50 ml-Kolben unter Stickstoff verdampft in ein mit dem gleichen Katalysator wie in den vorhergehenden Beispielen gefülltes Quarzrohr von 14 mm Durchmesser, das auf eine Länge von 16 cm in einem Röhrenofen auf 300 °C geheizt wird. Die Spaltprodukte werden in einer eingekühlten Vorlage gesammelt.

Nach Abdestillieren des gebildeten Ethanols erhält man durch Vakuumabdestillation 18 g (90% d.Th.) N-Vinyl-N-methyl-acetamid vom $Kp_{11}$ 51,5 °C.

Beispiel 5

Das wie unter Beispiel b) 8 erhaltene undestillierte Rohprodukt der Methylierungsreaktion wird an einer 1.20 m Füllkörperkolonne an der Wasserstrahlpumpe mit einem Rücklaufverhältnis von 1:8 langsam abdestilliert. Man erhält als Destillat N-Vinyl-N-methyl-acetamid.

Verfährt man in gleicher Weise mit einem nach Beispiel b) 7 hergestellten Methylierungsrohprodukt, das kein quartäres Ammoniumsalz enthält, so destilliert N-α-Methoxyethyl-N-methylacetamid unverändert über.

$b_1$) Alkoholabspaltung aus N-α-Alkoxyethyl-carbonsäureamiden

Beispiel 1

N-Vinylformamid

N-α-Methoxyethylformamid wird mit einem Durchsatz von 100 g/h in schwachem Stickstoffstrom bei 100 Torr aus einem auf 180 °C geheizten Rührkolben flach-abdestilliert in ein mit Quarzbruch gefülltes Quarzrohr von 25 mm Durchmesser, das auf eine Länge von 50 cm beheizt wird. Die Spaltprodukte werden in einem absteigenden Kühler kondensiert und in einer Vorlage gesammelt.

Die Tabelle zeigt die Versuchsergebnisse bei verschiedenen Reaktionstemperaturen.

Das Rohprodukt wird im Vakuum unter Zusatz von etwas Phenothiazin über eine 1,20 m – Vigreux-Kolonne rektifiziert.
$Kp_{0.2}$ : 41,5 °C $n_D^{20}$ 1,4923.

| Ausgangsmat. Durchsatz (g/h) | P (Torr) | Temp. (°C) Verdampfer | Reaktor | Rückstand (%) | Ausbeute (%) | Ausgangsmaterial i.Rohprod. (Mol %) |
|---|---|---|---|---|---|---|
| 100 | 100 | 180 | 510 | 3 | 94 | 7 |
| 100 | 100 | 180 | 420 | 3 | 92 | 7 |
| 100 | 100 | 180 | 350 | 3 | 88 | 18 |

Beispiel 2

N-Vinyl-acetamid

150 g (1,28 mol) N-α-Methoxyethylacetamid werden in 3 Stunden bei 190 Torr durch ein 30 cm langes und 1 cm weites Glasrohr destilliert, das mit Stahlwendeln gefüllt und auf 460–480 °C geheizt ist. In der auf −40 °C gekühlten Vorlage werden 143 g Rohprodukt aufgefangen. Daraus werden durch Destillation bei 0,2 Torr 103 g (119 Mol) kristallines N-Vinylacetamid (Siedepunkt

55 °C/0,2 Torr) gewonnen, das weniger als 1% Ausgangssubstanz enthält, entsprechend einer Ausbeute von 94% der Theorie.

$C_1$) Alkylierung von N-Vinylcarbonsäureamiden

Beispiel 1
N-Vinyl-N-methylformamid

Zu einer Mischung von

35,5 g (0,5 Mol) N-Vinylformamid und
78 g (0,55 Mol) Jodmethan tropft man bei 20 °C unter kräftigem Rühren innerhalb von 3 Stunden.
56 g (0,7 Mol) 50%ige Natronlauge. Nach weiteren 3 Stunden wird dekantiert und der wässrige Anteil mit Methylenchlorid extrahiert. Aus der organischen Phase erhält man nach Abziehen des Lösungsmittels durch Destillation i.V.
25 g (59 % d.Th.) N-Vinyl-N-methylformamid vom $Kp_{12}$ 42–45 °, dessen $^1H$–NMR-Spektrum identisch ist mit dem des Produktes von C Beispiel 2.

Beispiel 2
N-Vinyl-N-methylacetamid

Eine Mischung von

42,5 g (0,5 Mol) N-Vinylacetamid und
78 g (0,55 Mol) Jodmethan tropft man bei 20 °C in
320 g (4 Mol) kräftig gerührte 50%ige Natronlauge. 3 Std. nach beendetem Eintropfen dekantiert man und extrahiert den wässrigen Anteil mit Toluol. Aus der organischen Phase erhält man nach Abziehen des Lösungsmittels durch Destillation i.V. 32 g
32 g (65% d.Th.) N-Vinyl-N-methylacetamid vom $Kp_{11}$ 51,5 °C dessen $^1H$–NMR-Spektrum identisch ist mit dem des Produktes von C Beispiel 1.

**Patentansprüche:**

1. Verfahren zur Herstellung von N-Vinyl-N-alkyl-carbonsäureamiden der Formel I

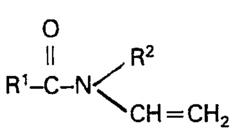

(I)

worin $R^1$ = H, $CH_3$ oder $C_2H_5$, vorzugsweise H oder $CH_3$, und $R^2$ = $CH_3$ oder $C_2H_5$, vorzugsweise $CH_3$, ausgehend von N-Ethyl-carbonsäureamiden der Formel II

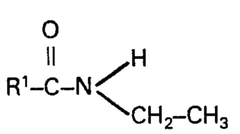

(II)

worin $R^1$ die gleiche Bedeutung wie in Formel I besitzt, gekennzeichnet durch die folgenden Verfahrensschritte:

a) Anodische Alkoxylierung der N-Ethyl-carbonsäureamide der Formel II mit Alkoholen der Formel III

$R^3OH$ (III)

worin $R^3$ = $C_1$-$C_4$–Alkyl, vorzugsweise $CH_3$ oder $C_2H_5$, insbesondere $CH_3$, zu N-α-Alkoxyethyl-carbonsäureamiden der Formel IV

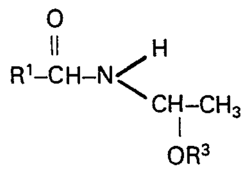

(IV)

worin $R^1$ und $R^3$ die gleiche Bedeutung wie in den Formeln I und III besitzen,

b) Alkylierung der N-α-Alkoxyethyl-carbonsäureamide IV durch Umsetzung mit einem Alkylierungsmittel der Formel V

$R^2X$ (V)

worin $R^2$ die gleiche Bedeutung wie in Formel I besitzt und

X = Cl, Br. J oder $SO_4/2$, in alkalischem Medium zu N-α-Alkoxyethyl-N-alkyl-carbonsäureamiden der Formel VI

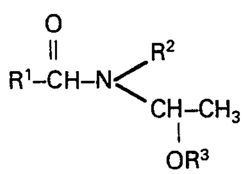

(VI)

worin $R^1$, $R^2$ und $R^3$, die bei den vorstehenden Formeln genannte Bedeutung besitzen, und

c) Abspaltung von Alkohol der Formel III aus den N-α-Alkoxyethyl-N-alkyl-carbonsäureamiden VI durch Erhitzen auf Temperaturen von etwa 60 bis 350 °C

oder – anstelle der Stufen b) und c) im Anschluss an Stufe a):

$b_1$) Abspaltung von Alkohol der Formel III aus den N-α-Alkoxyethyl-carbonsäureamiden IV zu N-Vinyl-carbonsäureamiden der Formel VII

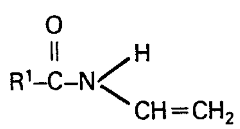

(VII)

worin $R^1$ die gleiche Bedeutung wie in Formel I besitzt, durch Erhitzen auf Temperaturen von etwa 60 bis 600 °C, und

$c_1$) Alkylierung der N-Vinyl-carbonsäureamide VII durch Umsetzung mit Alkylierungsmittel der Formel V in alkalischem Medium.

2. Verfahren nach Anspruch 1, dadurch gekennzeichnet, dass man die anodische Alkoxylierung der N-Ethyl-carbonsäureamide der Formel II mit Alkoholen der Formel III in Stufe a) in einer Elektrolysezelle mit glasartigem Kohlenstoff als Anodenmaterial und unter Verwendung von mindestens 1 Alkali- und/oder Tetraalkylammoniumalkosulfat – vorzugsweise von Tetramethylammoniummethosulfat – durchführt.

3. Verfahren nach den Ansprüchen 1 bis 2, dadurch gekennzeichnet, dass man als Alkylierungsmittel in den Stufen b) und $c_1$) Chlormethan oder -ethan, vorzugsweise Chlormethan, verwendet.

4. Verfahren nach den Ansprüchen 1 bis 3, dadurch gekennzeichnet, dass man während der Alkylierung in den Stufen b) und $c_1$) im Reaktionsgemisch immer einen Überschuss an Alkali- und Alkylierungsmittel aufrechterhält.

5. Verfahren nach den Ansprüchen 1 bis 4, dadurch gekennzeichent, dass man die alkalische Reaktion der Alkylierungsstufen b) und $c_1$) durch NaOH oder KOH, vorzugsweise NaOH, bewirkt.

6. Verfahren nach den Ansprüchen 1 bis 5, dadurch gekennzeichnet, dass man die Alkylierung in den Stufen b) und $c_1$) in Gegenwart von Phasentransferkatalysatoren, vorzugsweise von Tetraalkylammoniumsalzen, insbesondere von Tetraalkylammoniumchlorid oder -sulfat, durchführt.

7. Verfahren nach den Ansprüchen 1 bis 6, dadurch gekennzeichnet, dass man die Alkoholabspaltung in den Stufen c) und $b_1$) in Gegenwart von schwach sauren Katalysatoren durchführt.

**Revendications**

1. Procédé de préparation de N-vinyl-N-alkyl-carboxamides de formule I

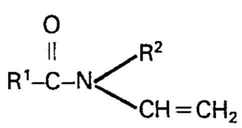

(I)

dans laquelle $R^1$ représente H, $CH_3$ ou $C_2H_5$, de préférence H ou $CH_3$, et $R^2$ représente $CH_3$ ou $C_2H_5$, de préférence $CH_3$, à partir de N-éthyl-carboxamides répondant à la formule II

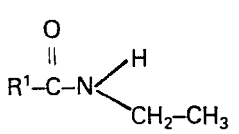

(II)

dans laquelle $R^1$ a la même signification que dans la formule I, procédé caractérisé par les étapes opératoires suivantes:

a) alcoxylation anodique des N-éthyl-carboxamides de formule II avec des alcools répondant à la formule III

$R^3OH$ (III)

dans laquelle $R^3$ représente un radical alkyle en $C_1$–$C_4$, de préférence $CH_3$ ou $C_2H_5$, plus spécialement $CH_3$, pour obtenir des N-(α-alcoxy-éthyl)-carboxamides répondant à la formule IV

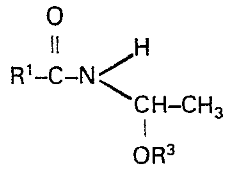

dans laquelle $R^1$ et $R^3$ ont les mêmes significations que dans les formules I et III;

b) alkylation des N-(α-alcoxy-éthyl)-carboxamides (IV) par réaction avec un agent d'alkylation répondant à la formule V

$R^2X$ (V)

dans laquelle $R^2$ a la même signification que dans la formule I et X représente Cl, Br, I ou $SO_4/2$, en milieu alcalin, réaction qui conduit à des N-(α-alcoxy-éthyl)-N-alkyl-carboxamides répondant à la formule VI

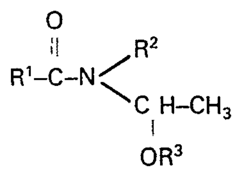

(VI)

dans laquelle $R^1$, $R^2$ et $R^3$ ont les significations qui ont été données ci-dessus à propos des formules précédentes, et c) enlèvement d'un alcool de formule III à partir des N-(α-alcoxy-éthyl)-N-alkyl-carboxamides (VI) par chauffage à des températures d'environ 60 à 350 °C,

ou, au lieu des étapes b) et c), à la suite de l'étape a): $b_1$) enlèvement d'un alcool de formule III à partir de N-(α-alcoxy-éthyl)-carboxamides (IV) pour obtenir des N-vinyl-carboxamides répondant à la formule VII

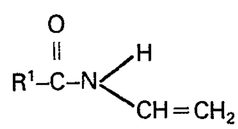

(VII)

dans laquelle $R^1$ a la même signification que dans la formule I, par chauffage à des températures d'environ 60 à 600 °C, et $c_1$) alkylation des N-vinyl-carboxamides (VII) par réaction avec un agent d'alkylation de formule V, en milieu alcalin.

2. Procédé selon la revendication 1, caractérisé en ce qu'on effectue l'alcoxylation anodique des N-éthyl-carboxamides de formule II avec des alcools de formule III, à l'étape a), dans une cellule d'électrolyse dont le matériau anodique est du carbone vitrifié, et avec emploi d'au moins un alkosulfate de métal alcalin et/ou de tétraalkylammonium, de préférence de méthosulfate de tétra-méthylammonium.

3. Procédé selon l'une des revendications 1 et 2, caractérisé en ce qu'on utilise, comme agent d'alkylation dans les étapes b) et $c_1$), le chloro-méthane ou le chloro-éthane, de préférence le chloro-méthane.

4. Procédé selon l'une quelconque des revendications 1 à 3, caractérisé en ce qu'au cours de l'alkylation, dans les étapes b) et $c_1$), on maintient toujours dans le mélange réactionnel un excès d'agent alcalin et d'agent d'alkylation.

5. Procédé selon l'une quelconque des revendications 1 à 4, caractérisé en ce que l'alcalinité dans les étapes d'alkylation b) et $c_1$) est créée par NaOH ou KOH, de préférence par NaOH.

6. Procédé selon l'une quelconque des revendications 1 à 5, caractérisé en ce que l'alkylation, dans les étapes b) et $c_1$), est effectuée en présence de catalyseurs de transfert de phase, qui sont de préférence des sels de tétra-alkyl-ammoniums, plus spécialement des chlorures ou des sulfates de tétra-alkyl-ammonium.

7. Procédé selon l'une quelconque des revendications 1 à 6, caractérisé en ce que la réaction d'enlèvement d'alcool, dans les étapes c et $b_1$), est effectuée en présence de catalyseurs légèrement acides.

**Claims:**

1. Process for the preparation of N-vinyl-N-alkyl-carboxylic acid amides of the formula I

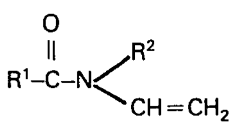 (I)

in which $R^1$=H, $CH_3$ or $C_2H_5$, preferably H or $CH_3$, and $R^2$=$CH_3$ or $C_2H_5$, preferably $CH_3$, starting from N-ethyl-carboxylic acid amides of the formula II

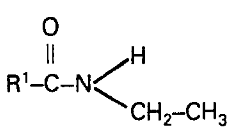 (II)

in which $R^1$ has the same meaning as in formula I, characterized by the following process steps:

a) anodic alkoxylation of the N-ethyl-carboxylic acid amides of the formula II with alcohols of the formula III

$R^3OH$ (III)

in which $R^3$ = ($C_1$–$C_4$)-alkyl, preferably $CH_3$ or $C_2H_5$ and in particular $CH_3$, to give N-α-alkoxyethyl-carboxylic acid amides of the formula IV

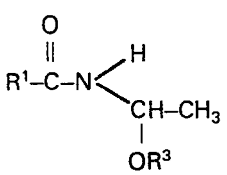 (IV)

in which $R^1$ and $R^3$ have the same meaning as in the formulae I and III,

b) alkylation of the N-α-alkoxyethyl-carboxylic acid amide IV by reaction with an alkylating agent of the formula V

$R^2X$ (V)

in which $R^2$ has the same meaning as in formula I and X=Cl, Br, I or $SO_4/2$, in an alkaline medium to give N-α-alkoxyethyl-N-alkyl-carboxylic acid amides of the formula VI

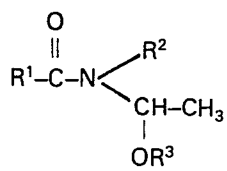 (VI)

in which $R^1$, $R^2$ and $R^3$ have the meanings given for the above formulae, and

c) splitting off of the alcohol of the formula III from the N-α-alkoxyethyl-N-alkyl-carboxylic acid amides VI by heating to temperatures of about 60 to about 350 °C, or instead of stages b) and c), after stage a)

$b_1$) splitting off of the alcohol of the formula III from the N-α-alkoxyethyl-carboxylic acid amides IV by heating to temperatures of about 60 to about 600 °C, to give N-vinyl-carboxylic acid amides of the formula VII

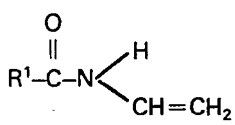 (VII)

in which $R^1$ has the same meaning as in formula I, and $c_1$) alkylation of the N-vinyl-carboxylic acid amides VII by reaction with an alkylating agent of the formula V in an alkaline medium.

2. Process as claimed in Claim 1, characterized in carrying out the anodic alkoxylation of the N-ethylcarboxylic acid amides of the formula II with alcohols of the formula III in stage a) in an electrolysis cell with vitreous carbon as the anode material, using at least one alkali metal alkosulfate and/or tetraalkylammonium alkosulfate, preferably tetramethylammonium methosulfate.

3. Process as claimed in Claims 1 to 2, characterized in using methyl or ethyl chloride, preferably methyl chloride, as the alkylating agent in stages b) and $c_1$).

4. Process as claimed in Claims 1 to 3, characterized in always maintaining excess of alkali and alkylating agent in the reaction mixture during the alkylation in stages b) and $c_1$).

5. Proces as claimed in Claims 1 to 4, characterized in producing the alkaline reaction of the alkylating stages b) and $c_1$) by NaOH or KOH, preferably NaOH.

6. Process as claimed in Claims 1 to 5, characterized in carrying out the alkylation in stages b) and $c_1$) in the presence of phase transfer catalysts, preferably tetraalkylammonium salts, in particular tetraalkylammonium chloride or sulfate.

7. Process as claimed in Claims 1 to 6, characterized in carrying out the splitting off of alcohol in stages c) and $b_1$) in the presence of weakly acid catalysts.